(19) Europäisches Patentamt
European Patent Office
Office européen des brevets

(11) Numéro de publication : **0 321 322 B1**

# (12) FASCICULE DE BREVET EUROPEEN

(45) Date de publication du fascicule du brevet : **21.11.91 Bulletin 91/47**

(51) Int. Cl.⁵ : **B01J 13/02,** A61K 9/50, C08G 77/06

(21) Numéro de dépôt : **88403095.8**

(22) Date de dépôt : **07.12.88**

## (54) Particules composites magnétisables à base d'organopolysiloxane réticulé, leur procédé de préparation et leur application en biologie.

(30) Priorité : **18.12.87 FR 8717718**

(43) Date de publication de la demande : **21.06.89 Bulletin 89/25**

(45) Mention de la délivrance du brevet : **21.11.91 Bulletin 91/47**

(84) Etats contractants désignés : **AT BE CH DE ES FR GB GR IT LI LU NL SE**

(56) Documents cités :
**EP-A- 0 252 858**
**DE-A- 3 224 484**
**FR-A- 2 429 616**
**US-A- 3 220 972**

(73) Titulaire : **RHONE-POULENC CHIMIE**
**25, quai Paul Doumer**
**F-92408 Courbevoie Cédex (FR)**

(72) Inventeur : **Leising, Frédéric**
**11, allée des Fauvettes La Condamine**
**F-69440 Mornant (FR)**
Inventeur : **Torres, Ghislaine**
**104, rue Ney**
**F-69006 Lyon (FR)**

(74) Mandataire : **Vignally, Noel et al**
**Rhône-Poulenc Chimie Service Brevets**
**Chimie Centre de Recherches de Saint-Fons**
**B.P. 62**
**F-69190 Saint-Fons Cédex (FR)**

**EP 0 321 322 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des particules composites magnétisables à base d'organopolysiloxane réticulé, se présentant telles quelles ou en dispersion aqueuse, leur procédé de préparation et leur application en biologie.

Des particules composites magnétisables constituées d'une matrice organique à base d'acroléine polymérisée sont déjà connues de la demande allemande DE-A 3324484.

Selon l'invention, il s'agit de particules composites magnétisables, se présentant telles quelles ou en dispersion aqueuse et constituées :

— d'une matrice à base d'un organopolysiloxane réticulé dérivé de l'hydrosilylation

• d'au moins un organopolysiloxane (appelé SiVi) contenant par molécule au moins deux groupes vinyles liés chacun à un atome de silicium, présentant une viscosité de l'ordre de 20 à 30.000.000 mPas à 25°C et portant éventuellement des motifs ionogènes et/ou réactifs non vinyliques liés à un atome de silicium ou à un atome de carbone d'un groupement hydrocarboné relié à la chaine organopolysiloxane par une liaison Si-C

• avec au moins un organohydrogénopolysiloxane (appelé SiH) contenant par molécule au moins trois atomes d'hydrogène liés chacun à un atome de silicium, présentant une viscosité de l'ordre de 5 à 1500 mPas à 25°C, de préférence entre 20 et 150 mPas à 25°C et portant éventuellement des motifs ionogènes et/ou réactifs non vinyliques liés à un atome de silicium ou à un atome de carbone d'un groupement hydrocarboné relié à la chaine organohydrogénopolysiloxane par une liaison Si-C

— et, encapsulées dans ladite matrice, de charges magnétisables présentant une taille généralement inférieure à 30 nm (300 Å), de préférence de l'ordre de 8-12 nm (80 à 120 Å), lesdites charges étant revêtues d'un agent dispersant non hydrosoluble.

Les organopolysiloxanes SiVi peuvent être représentés par la formule I suivante :

$$R'' R' R Si O (Si R R''' O)_n (Si R''' R' O)_m Si R R' R'' \quad (I)$$

formule dans laquelle :

• les symboles R sont identiques ou différents et représentent un radical alkyle en $C_1$-$C_4$, phényle, trifluoro -3,3,3 propyle ;

• les symboles R' sont identiques ou différents et représentent R ou un radical vinyle, le nombre de radicaux vinyles étant de 2 au moins par macromolécule ;

• les symboles R" sont identiques ou différents et représentent R ou un radical OH

• les symboles R"' sont identiques ou différents et représentent R ou un motif -r-X, où r représente un radical organique bivalent et X un groupe ionogène et/ou réactif non vinylique

• au moins 60% des radicaux représentés par les symboles R, R' et R" sont des radicaux méthyles.

• les symboles n et m peuvent être nuls séparement, R' représentant un radical vinyle si m est nul, et ont une valeur suffisante pour assurer une viscosité du polymère de 20 mPas à 30.000.000 mPas à 25°C et un nombre de motifs ionogènes et/ou réactifs non vinyliques éventuels allant de 1 à 1000, de préférence de l'ordre de 5 à 500 par molécule d'organopolysiloxane SiVi et d'organohydrogénopolysiloxane SiH.

A titre d'exemple d'organopolysiloxane SiVi, on peut citer :

— les polymères de formule II

$$(CH_2 = CH)R_2Si\text{-}O\text{-}(R R''' Si\text{-}O)_n\text{-}Si R_2(CH = CH_2) \quad (II)$$

où les symboles R et R"' ont la définition donnée ci-dessus, n ayant une valeur suffisante pour assurer une viscosité du polymère de 20 mPas à 30.000.000 mPas à 25°C

— et ceux de formule III

$$R''' R' R Si\text{-}O(R R''' Si\text{-}O)_n\text{-}[(CH_2 = CH)R''' Si\text{-}O\text{-}]_m\text{-}Si R R'R'' \quad (III)$$

où les symboles R, R', R" et R"' ont la signification donnée ci-dessus, n et m ayant une valeur suffisante pour assurer une viscosité du polymère de 20 mPas à 30.000.000 mPas à 25°C.

L'organoghydrogénopolysiloxane SiH peut être linéaire, ramifié ou cyclique.

Parmi les organohydrogénopolysiloxanes SiH préférentiels on peut citer ceux de formule IV :

$$Y R_2 Si O (R R''' Si O)_p(YR Si O)_q Si R_2 Y \quad (IV)$$

formule dans laquelle les symboles R sont identiques ou différents et ont la définition donné ci-dessus, avec au moins 80% de radicaux méthyles, le symbole Y représente R ou un atome d'hydrogène, le nombre d'atomes d'hydrogène étant de 3 au moins par molécule de polymère, le symbole R''' a la définition donnée ci-dessus, les symboles p et q étant tels que ledit polymère SiH présente une viscosité de l'ordre de 5 à 1500 mPas à 25°C, de préférence de l'ordre de 20 à 150 mPas à 25°C et un nombre de motifs ionogènes et/ou réactifs non vinyliques éventuels allant de 1 à 1000, de préférence de l'ordre de 5 à 500 par molécule d'organohydrogénopolysiloxane SiH et d'organopolysiloxane SiVi.

Parmi les radicaux organiques divalents pouvant constituer le radical r bivalent, on peut citer : les radicaux alkylènes linéaires ou ramifiés en $C_1$-$C_{18}$, éventuellement prolongés par 1 à 5 groupes bivalents éthylène amine, par 1 à 50 groupes oxyde d'alkylène en $C_1$-$C_3$, ou par un groupe

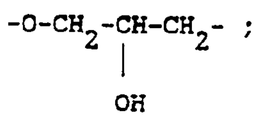

les radicaux polyoxyalkylènes contenant de 1 à 50 chaînons oxyalkylène en $C_1$-$C_3$.

A titre d'exemple de radicaux bivalents, on peut citer :

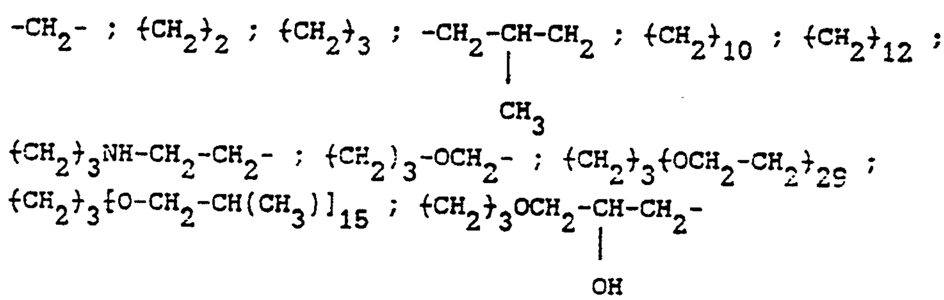

Parmi les groupements réactifs ou ionogènes X pouvant constituer le symbole X, on peut citer les groupements : époxy, hydroxy, carboxy, aldéhyde, ester, acétoester, mercapto, mercaptoester, mercaptoalcoxy, amino, alkylamino, dialkylamino, trialkylamino, ammonium quaternaire, aminoalcool, amido, hydrazide, hydrazino, halogénoalkyle en $C_1$-$C_3$, halogénobenzyle, cyano, cyanato,

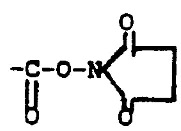 , :

sulfate, sulfonyle.

Les polymères SiVi et SiH ne portant de motifs ionogènes et/ou réactifs non vinyliques sont bien connus. Ils sont décrits par exemple dans les brevets américains n° 3220972 n° 3344111 et 3436366.

Les polymères SiVi et SiH portant des groupes ionogènes et/ou réactifs non vinyliques peuvent être préparés selon des méthodes bien connues.

Ainsi les polymères SiVi portant des groupes ionogènes et/ou réactifs non vinyliques peuvent être obtenus par :

— équilibrage d'un cyclotétrasiloxane et d'un cyclotétrasiloxane vinylé en présence d'un disiloxane fonctionnalisé

— équilibrage d'un cyclotétrasiloxane fonctionnalisé en présence d'un disiloxane divinylé

— équilibrage d'une huile polysiloxane fonctionnalisée en présence d'un divinylsiloxane et/ou d'un cyclotetrasiloxane

...

Les polymères SiH portant des groupes ionogènes et/ou réactifs non vinyliques peuvent être obtenus par exemple par :

— équilibrage d'un cyclotétrasiloxane et d'une huile polysiloxane à fonctions Si H internes en présence

d'un disiloxane fonctionnalisé non réactif vis-à-vis des groupements SiH

— équilibrage d'un cyclotétrasiloxane fonctionnalisé (non réactif vis-à-vis de groupements SiH) en présence d'un dihydrogénodisiloxane

— équilibrage d'une huile polysiloxane fonctionnalisée (non réactive vis-à-vis de groupements SiH) en présence d'un dihygénodisiloxane ou d'un polysiloxane à fonctions SiH internes

— ...

Pour une bonne réalisation de l'invention, la matrice à base de polyorganosiloxane réticulé dérive de l'hydrosilylation d'au moins un organopolysiloxane SiVi et d'au moins un organohydrogénopolysiloxane SiH, avec des quantités relatives des deux types de polymère telles que le rapport en nombre des "groupes SiH" (atome d'hydrogène lié à un atome de silicium) sur les "groupes SiVi" (groupe vinyle lié à un atome de silicium) soit compris entre 0,75/1 et 4/1, de préférence entre 0,75/1 et 1,5/1.

Parmi les matériaux pouvant constituer les charges magnétisables encapsulées dans la matrice de polyorganosiloxane, on peut citer la magnétite, l'hématite, le dioxyde de chrome, les ferrites telles que les ferrites de manganèse, nickel, manganèse-zinc..., les alliages de cobalt, nickel, gadolinium, samarium-cobalt... Les matériaux préférentiels sont la magnétite et la l'hématite.

La quantité de charges magnétisables encapsulées dans la matrice de polyorganosiloxane correspond à environ 0,5 à 50% en poids desdites charges par rapport au poids de matrice et de préférence de 0,5 à 35% en poids.

Parmi les agents dispersants formant un revêtement non hydrosoluble autour des charges magnétisables on peut citer les acides gras, les amines, les amides... contenant au moins 12 atomes de carbone, et plus particulièrement les acides gras contenant environ 18 atomes de carbone, tels que les acides oléiques, linoléiques et linoléniques.

Sont également particulièrement intéressants les agents dispersants siliconés tels que ceux de formule V :

$$R_3\,Si\,O\,(R_2\,Si\,O)_x\,(R_2\,Si)_y\,Z \quad (V)$$

formule dans laquelle :

- les symboles R ont la définition donnée ci-dessus, et représentent de préférence le radical méthyle
- x est un nombre entier allant de 0 à 1000
- y est un nombre entier égal à 0 ou 1
- Z représente une fonction coordinable avec les charges magnétisables telle qu'un radical hydroxyle, SH, $NH_2$..., alkyle substitué par un groupe fonctionnel comme par exemple les radicaux $-(CH_2)_3\,NH_2$ ; $-(CH_2)_3\,SH$ ; $-(CH_2)_3-NH-C_2H_5$ ; $-(CH_2)_3\,NH-(CH_2)_2\,NH_2$ ; $-O-CO-CH_2-COCH_3$ ;

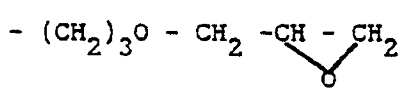 ; $-(CH_2)_3-S-CH_2-COOH$,

$-(CH_2)_3-O-CO-CH_2-SH$ ; $-CH_2-COOH$...

Les particules magnétisables faisant l'objet de l'invention peuvent être de taille uniforme ou présenter une granulométrie étalée ; leur diamètre peut être de l'ordre de 0,05 à 3 µm et généralement de l'ordre de 0,2 à 2 µm.

Elles peuvent se présenter telles quelles ou en dispersion dans l'eau ; la quantité de particules magnétisables à l'état dispersé dans l'eau peut correspondre à environ 10 à 70% en poids par rapport au poids total de dispersion et généralement de l'ordre de 15 à 50% en poids.

La présente invention a également pour objet un procédé de préparation desdites particules composites magnétisables.

Le procédé consiste :

1) à introduire dans un milieu aqueux contenant au moins un tensio-actif, un mélange :

- d'une solution d'au moins un organopolysiloxane SiVi tel que défini ci-dessus dans un solvant organique de point d'ébullition inférieur à 100°C ou susceptible de former avec l'eau un azéotrope de point d'ébullition inférieur à 100°C et de préférence inférieur à 95°C
- d'une solution d'au moins un organohydrogénopolysiloxane SiH tel que défini ci-dessus dans un solvant organique de point d'ébullition inférieur à 100°C ou susceptible de former avec l'eau un azéotrope de point d'ébullition inférieur à 100°C et de préférence inférieur à 95°C
- et d'un fluide magnétique constitué de particules magnétisables de taille généralement inférieure à 30 nm (300 Å) et de préférence de l'ordre de 8-12 nm (80 à 120 Å) en suspension dans un liquide vecteur

organique solvant desdits polymères SiVi et SiH et de point d'ébullition inférieur à 100°C ou susceptible de former avec l'eau un azéotrope de point d'ébullition inférieur à 100°C et de préférence inférieure à 95°C

2) à homogénéiser le milieu obtenu

3) à effectuer une opération d'hydrosilylation en émulsion aqueuse des polymères SiH et SiVi

4) à éliminer les solvants organiques et le liquide vecteur organique

5) et éventuellement à éliminer l'eau.

Parmi les solvants des polymères SiVi et SiH pouvant être mis en oeuvre, on peut citer le cyclohexane, le chlorure de méthylène, le benzène, l'hexane, l'octane, le toluène, le tétrachlorure de carbone...

Le solvent est mis en oeuvre afin d'obtenir une viscosité des solutions inférieure à 1.000 mPas à 25°C, de préférence inférieure à 500 mPas. Il est évident qu'un solvant peut ne pas être nécessaire lorsque le poids moléculaire d'un ou des polymères est suffisamment bas.

Les fluides magnétiques sont appelés couramment "ferrofluides". Ce sont des suspensions colloïdales extrêmement stables de charges ferro ou ferrimagnétiques de dimension inférieure au micron, dans un liquide vecteur ; ils restent fluides en présence des champs magnétiques externes.

Ce type de matériaux pouvant constituer les charges ferro ou ferrimagnétiques a déjà été mentionné ci-dessus ; les matériaux préférentiels sont la magnétite et l'hématite.

Selon l'invention, le liquide vecteur peut être tout liquide organique solvant des polymères SiVi et SiH ou susceptible de former avec l'eau un azéotrope de point d'ébullition inférieur à 100°C, et présentant un point d'ébullition inférieur à 100°C. Tout en étant du même type, le liquide vecteur du fluide magnétique utilisé peut être semblable ou différent du solvant des polymères mis en oeuvre.

Ledit fluide magnétique peut être préparé d'une manière connue par peptisation dans un agent dispersant de grains de charges magnétisables obtenus par broyage ou par précipitation par voie sol-gel, puis dispersion des grains peptisés dans un liquide vecteur organique. Les agents dispersants utiles dans la préparation du fluide magnétique peuvent être des acides gras, des amines, des amides... contenant au moins 12 atomes de carbone, et plus particulièrement les acides gras contenant environ 18 atomes de carbone, tels que les acides oléiques, linoléiques et linoléniques, ainsi que les dispersants siliconés de formule V cités ci-dessus.

La concentration dudit fluide magnétique en charges magnétisables peut être de l'ordre de 20 à 60% en poids et généralement de l'ordre de 30 à 60% en poids.

La quantité de fluide magnétique mise en oeuvre pour réaliser le procédé faisant l'objet de l'invention est telle que le poids de charges magnétisables dudit fluide magnétique corresponde à environ 0,5 à 50% du poids des polymères SiVi et SiH, de préférence de 0,5 à 35%.

Le milieu aqueux dans lequel est introduit le mélange de solutions de polymères SiVi et SiH et de fluide magnétique, peut présenter une concentration en poids de tensio-actif de l'ordre de 0,5 à 15%, de préférence de l'ordre de 1 à 10%.

Le tensio-actif contenu dans le milieu aqueux dans lequel est introduit le mélange de solutions de polymères et de fluide magnétique peut être tout émulsifiant de type non-ionique, anionique ou cationique hydrosoluble ou susceptible de former des micelles dans l'eau.

Parmi les tensio-actifs non-ioniques, on peut citer les alcools gras polyéthoxylés, les alkylphénols polyéthoxylés, les acides gras polyéthoxylés, les condensats d'oxydes d'éthylène et de propylène, les amides gras polyéthoxylés, les amines grasses polyéthoxylées, les esters d'acides gras, les éthanolamides, l'alcool polyvinylique...

Parmi les tensio-actifs anioniques, on peut citer les alkylsulfates, alkylsulfonates, alkylarylsulfonates, sulfosuccinates, sulfosuccinates de sodium...

Parmi les tensio-actifs cationiques, on peut citer les halogénures d'amines grasses, les halogénures, sulfates, méthylsulfates, acétates d'amines grasses éthoxylées, les halogénures d'ammonium quaternaire en $C_{10}$-$C_{18}$...

La quantité de milieu aqueux pouvant être mise en oeuvre est telle que la quantité de tensio-actif corresponde à environ 0,5 à 60% en poids, de préférence de l'ordre de 1 à 50% en poids par rapport au poids de polymères SiVi et SiH.

L'operation d'introduction dans le milieu aqueux du mélange solutions de polymères SiVi et SiH/fluide magnétique est réalisée progressivement sous agitation à température ordinaire (de l'ordre de 15 à 40°C).

L'opération d'homogénéisation est réalisée en une ou plusieurs étapes à une température de l'ordre de 20 à 60°C, à l'aide d'un système d'agitation énergique tel que moulin à colloïdes, pompe à haute pression, agitateur à vibrations, appareil à ultra-sons... jusqu'à obtenir une dispersion de gouttelettes de la phase organique contenant les charges magnétisables, gouttelettes de taille comprise entre environ 0,065 à 3,2 µm et de préférence de 0,35 à 2,2 µm. Lesdites gouttelettes sont constituées de polymères SiVi et SiH gonflés de solvant(s) et renfermant les charges magnétisables.

L'opération de réticulation des polymères SiVi et SiH est réalisée à l'aide d'une quantité efficace d'un catalyseur d'hydrosilylation.

On peut utiliser comme catalyseur des composés d'un métal du groupe du platine, en particulier leurs sels et complexes notamment les complexes de platine-oléfine comme décrit dans les brevets US-A 3159601 et 3159662, les produits de réaction des dérivés du platine avec des alcools, des aldéhydes et des éthers décrits dans le brevet US-A-3220972, les catalyseurs platine-vinylsiloxane décrits dans les brevets français FR-A 1313846 et son addition 88676 et le brevet français FR-A 1480409 ainsi que les complexes décrits dans les brevets US-A 3715334, 3775452 et 3814730, ainsi qu'un catalyseur au rhodium tel que décrit dans les brevets US-A 3296291 et 3928629.

Les métaux préférés du groupe de platine sont le platine et le rhodium ; le ruthénium bien que moins actif mais moins cher, est également utilisable.

La quantité de catalyseur mise en oeuvre est généralement de l'ordre de 5 à 100 ppm, de préférence de 10 à 60 ppm, de catalyseur calculée en poids de métal par rapport au poids total des polymères SiVi et SiH.

Le catalyseur est introduit dans le milieu de réticulation sous forme d'une émulsion aqueuse.

Pour préparer cette émulsion aqueuse on passe dans un broyeur à colloïdes le catalyseur avec un émulsifiant du même type que celui mis en oeuvre pour préparer la solution de surfactant décrite ci-dessus et de l'eau.

L'émulsion de catalyseur est introduite dans le milieu de réticulation obtenu après homogénéisation du mélange des solutions de polymères SiVi et SiH/fluide magnétique/eau et élimination des solvants organiques et liquide vecteur organique.

Le milieu est alors chauffé à une température de l'ordre de 20 à 70°C et de préférence de l'ordre de 20 à 40°C.

Cette opération peut durer de l'ordre de 15 mn à 4 h, de préférence de 15 à 30 mn.

On obtient ainsi une dispersion aqueuse contenant de 10 à 60%, de préférence de 15 à 50% de son poids de particules composites de dimension pouvant aller de 0,05 à 3 μm, de préférence de 0,2 à 2 μm et constituées d'une matrice à base de polyorganosiloxane réticulé (le taux de réticulation pouvant aller de 40 à 100% et généralement de 90 à 100%), et, encapsulées dans ladite matrice, de charges magnétisables de dimension inférieure à 30 nm (300 Å) et de préférence de l'ordre de 8-12 nm (80 à 120 Å).

Le poids de particules magnétisables en dispersion aqueuse peut être ajusté à volonté, soit par élimination partielle de l'eau après aimantation, soit par élimination totale de l'eau après aimantation et ajout d'eau déionisée jusqu'à obtenir un taux d'extrait sec de l'ordre de 10 à 70% en poids et de préférence de l'ordre de 15 à 50% en poids.

Le ou les solvants des polymères et le liquide vecteur sont ensuite éliminés par distillation sous vide.

Selon une variante de réalisation le ou les solvants des polymères et le liquide vecteur sont éliminés par distillation sous vide juste avant l'opération de réticulation. Dans ces conditions, l'opération de réticulation est réalisée pendant plus de 2 heures ; généralement cette opération dure 4 heures environ.

Si désiré, les particules peuvent, bien entendu, être séparées du milieu par simple aimantation.

Les particules magnétisables faisant l'objet de l'invention présentent des particularités qui les rendent notamment intéressantes en biologie.

En effet, les particules magnétisables de l'invention présentent les avantages suivants :

- elles sont stérilisables à chaud, pendant 2 heures à 122°C, ce qui signifie qu'elles restent actives après stérilisation ;
- les charges magnétisables qu'elles renferment sont enrobées par la matrice de silicone, ce qui évite toute interaction desdites charges avec le milieu réactionnel dans lequel les particules sont utilisées ;
- elles sont biotolérantes et non toxiques, ce qui leur permet de ne pas perturber les processus biologiques in vitro et de pouvoir être utilisées in vivo ;
- elles sont magnétisables, ce qui leur permet d'être séparées par simple aimantation du milieu réactionnel dans lequel elles ont été utilisées ; les opérations de lavage sont également de ce fait accélérées.

Lesdites particules magnétisables peuvent être utilisées par exemple comme supports actifs :

- d'anticorps ou d'antigènes pour les tests de diagnostiques, les séparations par affinité des composés biologiques ; la fixation des molécules biologiques peut, si nécessaire, être réalisée par des méthodes de couplage bien connues faisant intervenir des agents de couplage (glutaraldéhyde, carbodiimide hydrosoluble), ou bien consistant à activer les fonctions éventuelles du polyorganosiloxane (par exemple par diazotation, par action du bromure de cyanogène, de l'hydrazine...) et à faire réagir la molécule à fixer, etc...
- de systèmes enzymatiques pour réactions biologiques
- de fixation de cultures cellulaires
- de médicaments ou de substances de révélation pour guider ceux-ci ou celles-ci in vitro ou in vivo vers le point de traitement choisi

• de molécules chimiques permettant une croissance de ces molécules par enchaînement rapide de réactions particulières comme la synthèse peptidique
• de groupements chimiques catalyseurs de réaction
• de groupements chimiques pour la séparation ou l'extraction de métaux ou d'isomères optiques.

Les exemples suivants sont donnés à titre indicatif et ne peuvent être considérés comme une limite du domaine et de l'esprit de l'invention.

Les ferrofluides mis en oeuvre pour réaliser les exemples 1-16 ont été préparés selon le mode opératoire générale suivant :

On dissout successivement dans 31 kg d'eau 11 kg de $FeCl_3$, $6H_2O$, puis 7,5 kg de $FeSO_4$, $7H_2O$. La solution obtenue est introduite dans un réacteur contenant 20 kg d'une solution aqueuse à 20% d'ammoniaque. Le réacteur est porté à 60°C et maintenu à cette température pendant 15 minutes ; on ajoute 2,4 kg d'acide oléique et l'on maintient le milieu sous agitation à 60°C pendant 15 minutes ; le milieu est refroidi à 25°C puis neutralisé par de l'acide chlorhydrique à 38% jusqu'à obtenir un pH de 5,5.

Après filtration sous vide, le produit est lavé à l'eau, puis à l'acétone et séché. Le produit est repris par un solvant organique (liquide vecteur) puis l'eau résiduelle éliminée par distillation azéotropique.

La quantité de liquide vecteur est telle que la concentration en magnétite formée est de 50% en poids.

La taille des charges de magnétite, mesurée par microscopie électronique est de l'ordre de 10 nm (100 Å).

EXEMPLE 1

13 g d'un ferrofluide dont le liquide vecteur est le cyclohexane (ce qui correspond donc à 6,5 g de magnétite) sont introduits dans une solution constituée de :

— 400 g de cyclohexane
— 31 g d'huile 50620 contenant 3% en poids de groupes vinyles, huile commercialisée par RHONE-POULENC (il s'agit d'une huile polydiméthylsiloxane contenant des groupes SiVi le long de la chaîne macromoléculaire ainsi qu'aux extrêmités de celles-ci)
— et de 4 g d'huile 628 V 30 H 10 commercialisée par RHONE-POULENC (il s'agit d'une huile polydiméthylsiloxane portant des groupes SiH en quantité correspondant à 1% en poids d'hydrogène par rapport à l'huile)

ce qui correspond à un rapport des concentrations en groupes réactionnels [Si H]/[Si Vi] voisin de 1.

Les caractéristiques des deux huiles sont les suivantes :

- huile Si Vi degré de polymérisation moyen = 300
viscosité = 300-500 mPa s à 25°C
- huile SiH degré de polymérisation moyen = 100
viscosité = 50 mPa s à 25°C

Le mélange obtenu est ensuite homogénéisé en présence de 800 g d'eau et 18,2 g d'émulsifiant CEMULSOL ON 10-20 à 86,5% de matière active commercialisée par la SOCIETE FRANCAISE D'ORGANO SYNTHESE (ce qui correspond à 45% en poids par rapport aux huiles silicones), dans une cuve à ultra-sons (appareil SONIFIER B-30 commercialisé par BRANSON SONIC POWER CO) pendant 10 minutes.

L'émulsion ainsi obtenue présente une granulométrie moyenne (mesure faite au COULTER NANOSIZER PSM série 17, commercialisé par Coulter Electronics Ltd) de 0,9-1 µm.

On introduit ensuite une émulsion du catalyseur 70889 (commercialisé par RHONE-POULENC, contenant 5% en poids de platine complexé) en quantité correspondant à 1/1000 en poids de platine par rapport au mélange d'huiles silicones. Le milieu est maintenu à 40°C pendant 4 heures.

Le cyclohexane est ensuite éliminé à 40°C sous pression réduite (175 mbar). Les dernières traces de cyclohexane sont éliminées par distillation azéotropique avec de l'acide acétique.

L'analyse chromatographique en phase gazeuse de la teneur en cyclohexane résiduel révèle des taux inférieurs à 15 ppm.

On obtient ainsi une dispersion de particules dont le diamètre moyen est de 0,7-0,8 µm et renfermant 18,5% en poids de charges magnétisables d'une taille de l'ordre de 10 nm (100 Å).

L'évaluation du degré de réticulation des particules, réalisée par extraction par solvant, révèle des taux de l'ordre de 95%.

L'encapsulation des charges magnétisables est constatée de la manière suivante :

100 mg de dispersion de particules sont introduits dans 100 ml d'acétone ; le précipité obtenu séparé et séché est additionné de 10 $cm^3$ d'acide chlorhydrique 1 N. Après 24 heures, on constate qu'il n'y a pas de coloration ni de la phase aqueuse ni de la surface des particules.

Les particules peuvent être séparées de la dispersion à l'aide d'un simple aimant de laboratoire. De même, la dispersion de particules peut être lavée par séparation par aimantation des particules et remplacement de la phase liquide par une solution aqueuse de CEMULSOL ON 10-20 à 1% de matière active.

EXEMPLE 2

On répète l'opération décrite à l'exemple 1 à partir :
— 13 g dudit ferrofluide, ce qui correspond à 6,5 g de pigment magnétisable
— d'une solution de :
- 400 g de cyclohexane
- 31 g d'huile 50620
- 4 g d'une huile polydiméthylsiloxane epoxydée portant des groupes SiH, de formule

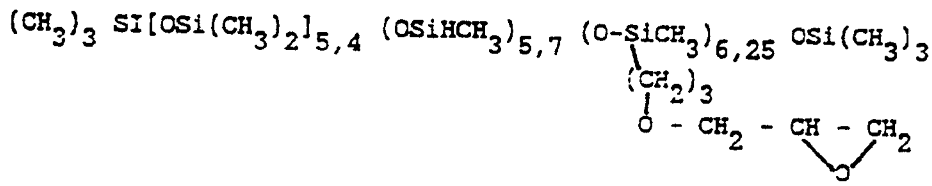

dont les caractéristiques sont les suivantes :
$\overline{Mn}$ = 2000
Concentration en groupes époxydes = 2,29 milliéquivalents/g d'huile
Concentration en groupes Si H = 2,10 milliéquivalents/g d'huile

L'homogénéisation est réalisée en présence de 800 g d'eau et 15,75 g de laurylsulfate de sodium ; la granulométrie moyenne de l'émulsion est de 0,9 µm.

Après réticulation on obtient une dispersion de particules dont le diamètre moyen est de 0,85 µm et renfermant 18,5% en poids de charges magnétisables d'une taille de l'ordre de 10 nm (100 Å).

Le taux de réticulation est de 97%.

Aimantation à saturation (mesure effectuée à l'aide d'un hystérésimètre sous 2786 $10^2$ A/m = 6,8 u e m/g).

EXEMPLES 3-9

On répète l'opération décrite à l'exemple 2 en réalisant l'opération d'homogénéisation selon les conditions indiquées au tableau I.

Les caractéristiques des dispersions de particules obtenues après réticulation dans les conditions décrites à l'exemple 2 figurent aux tableaux I et II.

EXEMPLE 10

On répète l'opération décrite à l'exemple 2 :
— en remplaçant l'huile 50620 par 31 g d'huile 621 V 20 commercialisée par RHONE-POULENC ; il s'agit d'une huile polydiméthylsiloxane α, ω divinylée dont les caractéristiques sont les suivantes :
$\overline{Mn}$ = 1800
Concentration en groupes Si Vi = 1,07 milliéquivalent/g d'huile
— en effectuant l'opération de réticulation à 20°C pendant 1 heure.

Les caractéristiques de la dispersion de particules obtenue figurent au tableau II.

EXEMPLE 11

On réalise l'opération décrite à l'exemple 2 en mettant en oeuvre :
— 22,5 g dudit ferrofluide, ce qui correspond à 11,25 g de pigment magnétisable.

L'homogénéisation est réalisée en présence de 800 g d'eau et de 18,2 g de CEMULSOL ON 10-20 à 86,5% de matière active ; la granulométrie moyenne de l'émulsion est de 0,85 µm.

Aprés réticulation dans les conditions décrites à l'exemple 1 ou 2, on obtient une dispersion de particules

dont le diamètre moyen est de 0,75 micron et renfermant 32,1% en poids de charges magnétisables d'une taille de l'ordre de 10 nm (100 Å).

Le taux de réticulation est de 97%.

Aimantation à saturation = 24 u e m/g.

EXEMPLE 12

On réalise l'opération décrite à l'exemple 2 en mettant en oeuvre :

— 43,4 g dudit ferrofluide, ce qui correspond à 21,7 g de pigment magnétisable.

L'homogénéisation est réalisée en présence de 800 g d'eau et de 18,2 g de CEMULSOL ON 10-20 à 86,5% de matière active ; la granulométrie moyenne de l'émulsion est de 1,2 µm.

Après réticulation dans les conditions décrites dans l'exemple 1 ou 2, on obtient une dispersion de particules dont le diamètre moyen est de 0,75 µm et renfermant 62% en poids de charges magnétisables.

Le taux de réticulation est de 92%.

Aimantation à saturation = 27 u e m/g.

EXEMPLE 13

On répète l'opération décrite à l'exemple 1 à partir de :

— 13 g dudit ferrofluide, ce qui correspond à 6,5 g de pigment magnétisable.

— d'une solution de :

- 400 g de cyclohexane
- 31 g d'huile 50620
- 4 g d'une huile polydiméthylsiloxane carboxyméthylée portant des groupes Si H, de formule

$$(CH_3)_3\ Si[OSi(CH_3)_2]_8\ (OSiHCH_3)_3\ (O\text{-}SiCH_3)_{5,5}\ OSi(CH_3)_3$$

$$|$$

$$(CH_2)_{12}$$

$$|$$

$$COO\ Me$$

dont les caractéristiques sont les suivantes :

$\overline{Mn}$ = 2400

Concentration en groupes Si H = 1,25 milliéquivalent/g d'huile

Concentration en groupes carboxyméthylés : 2,45 milliéquivalents/g d'huile.

L'homogénéisation est réalisée en présence de 800 g d'eau et 18,2 g de CEMULSOL ON 10-20 à 86,5% de matière active ; la granulométrie moyenne de l'émulsion est de 0,9 µm.

Après réticulation, on obtient une dispersion de particules dont le diamètre moyen est de 0,7 µm et renfermant 18,5% en poids de charges magnétisables.

Le taux de réticulation est de 98%.

Aimantation à saturation = 6,7 u e m/g

EXEMPLE 14

On répète l'opération décrite à l'exemple 1 à partir de :

— 13 g dudit ferrofluide, ce qui correspond à 6,5 g de pigment magnétisable

— d'une solution de

- 400 g de cyclohexane
- 31 g d'huile 50620
- 4 g d'une huile polydiméthylsiloxane carboxyméthylée portant des groupes Si H de formule

$$(CH_3)_3\ Si[OSi(CH_3)_2]_{81}\ (OSiHCH_3)_{18}\ (O\text{-}SiCH_3)_{132}\ OSi(CH_3)_3$$

$$|$$

$$(CH_2)_{12}$$

$$|$$

$$COO\ Me$$

dont les caractéristiques sont les suivantes :
$\overline{Mn}$ = 15.000
Concentration en groupes SiH : 1,25 milliéquivalent/g d'huile
Concentration en groupes carboxyméthylés : 8,8 milliéquivalents/g d'huile.

L'homogénéisation est réalisée en présence de 800 g d'eau et de 15,75 de chlorure de lauryltriméthylammonium.

La granulométrie moyenne de l'émulsion est de 1 µm.

Aprés réticulation, on obtient une dispersion de particules dont le diamètre moyen est de 0,8 micron et renfermant 18,5% en poids de charges magnétisables d'une taille de l'ordre de 10 nm (100 Å).

Le taux de réticulation est de l'ordre de 32%.

## EXEMPLE 15

On répète l'opération décrite à l'exemple 1 à partie de :

— 13 g dudit ferrofluide, ce qui correspond à 6,5 g de pigment magnétisable
— d'une solution de
  - 400 g de cyclohexane
  - 31 g d'huile 50620
  - 4 g d'une huile polydiméthylsiloxane epoxydée portant des groupes Si H de formule

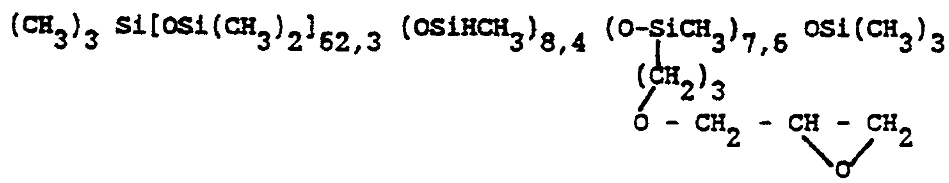

présentant une masse moléculaire en nombre $\overline{Mn}$ = 6.600.

L'homogénéisation est réalisée en présence de 800 g d'eau et 18,2 g d'émulsifiant CEMULSOL ON 10-20; la granulométrie moyenne de l'émulsion est de 0,95 µm.

Après réticulation on obtient une dispersion de particules dont le diamètre moyen est de 0,7 µm et renfermant 18,5% de charges magnétisables.

Le taux de réticulation est de 91%.

## EXEMPLE 16

On répète l'opération décrite à l'exemple 1 à partir de :

— 13 g dudit ferrofluide, ce qui correspond à 6,5 g de pigment magnétisable
— d'une solution de
  - 800 g de cyclohexane
  - 31 g de Gomme 789 commercialisée par RHONE-POULENC ; il s'agit d'un polydiméthylsiloxane contenant 700 ppm de groupes Si Vi le long de la chaine moléculaire et présentant les caractéristiques suivantes : viscosité : 13.000.000 m Pas
    degré de polymérisation moyen : 400.000
  - et 4 g d'une huile polydiméthylsiloxane époxydée portant des groupes Si H de formule

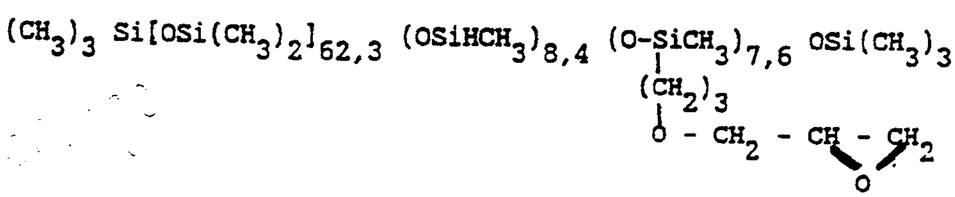

présentant une masse moléculaire en nombre $\overline{Mn}$ = 6.600.

L'homogénéisation est réalisée en présence de 400 g d'eau et 9 g de CEMULSOL ON 10-20 ; la granulométrie moyenne de l'émulsion est de 1 µm.

Après réticulation pendant 4 heures à 40°C on obtient une dispersion de particules dont le diamètre moyen est de 0,8 µm et renfermant 18,5% de charges magnétisables.

Le taux de réticulation est de 98%.

TABLEAU I

| EXEMPLE | EMULSIFIANT NATURE | QUANTITE g | Ø MICRON EMULSION | Ø MICRON PARTICULES | RETICULATION % | AIMANTATION u e m / g |
|---|---|---|---|---|---|---|
| 1 | ON 10-20 | 15,75 | 0,9 -1 | 0,7 - 0,8 | 95 | / |
| 2 | Na LS | 15,75 | 0,9 | 0,85 | 97 | 6,8 |
| 3 | Na DBS | 15,75 | 1 | 0,85 | 98 | 6,9 |
| 4 | K 30 | 15,75 | 1,1 | 0,75 | 99 | 6,7 |
| 5 | KL | 15,75 | 1,3 | 0,65 | 97 | 6,1 |
| 6 | LTMA | 15,75 | 0,9 | 0,75 | 98 | 8 |
| 7 | ON 10-20 | 15,75 | 0,7 | 0,6 | 97 | 11,3 |
| 8 | ON 10-20 | 10,5 | 1 | 0,5 | 93 | 9 |

ON 10-20 = CEMULSOL ON 10-20 exprimé en matière sèche
Na LS = lauryl sulfate de sodium
K 30 = alkylsulfonate de Na en $C_{13}$-$C_{17}$ commercialisé par BAYER
KL = laurate de potassium
LTMA = chlorure de lauryltriméthylammonium

TABLEAU II

| EXEMPLE | EMULSIFIANT NATURE | EMULSIFIANT QUANTITE g | Ø MICRON EMULSION | Ø MICRON PARTICULES | RETICULATION % | AIMANTATION u e m / g |
|---|---|---|---|---|---|---|
| 9 | ON 10-20 | 5,25 | 1,2 | 0,8 | 94 | 9 |
| 10 | ON 10-20 | 15,75 | 1 | 0,8 | 97 | 5,6 |
| 11 | ON 10-20 | 15,75 | 0,85 | 0,75 | 97 | 24 |
| 12 | ON 10-20 | 15,75 | 1,2 | 0,75 | 92 | 27 |
| 13 | ON 10-20 | 15,75 | 0,9 | 0,7 | 98 | 6,7 |
| 14 | LTMA | 15,75 | 1 | 0,8 | 32 | |
| 15 | ON 10-20 | 15,75 | 0,95 | 0,7 | 91 | |
| 16 | ON 10-20 | 7,78 | 1 | 0,8 | 98 | |

ON 10-20 = CEMULSOL ON 10-20 exprimé en matière sèche
LTMA = chlorure de lauryltriméthylammonium

**Revendications**

1. Particules composites magnétisables, se présentant telles quelles ou en dispersion aqueuse et constituées :

— d'une matrice à base d'un organopolysiloxane réticulé dérivé de l'hydrosilylation

• d'au moins un organopolysiloxane (appelé SiVi) contenant par molécule au moins deux groupes vinyles liés chacun à un atome de silicium, présentant une viscosité de l'ordre de 20 à 30.000.000 mPas à 25°C et portant éventuellement des motifs ionogènes et/ou réactifs non vinyliques liés à un atome de silicium ou à un atome de carbone d'un groupement hydrocarboné relié à la chaine organopolysiloxane par une liaison Si-C

• avec au moins un organohydrogénopolysiloxane (appelé SiH) contenant par molécule au moins trois atomes d'hydrogène liés chacun à un atome de silicium, présentant une viscosité de l'ordre de 5 à 1500 mPas à 25°C, et portant éventuellement des motifs ionogènes et/ou réactifs non vinyliques liés à un atome de silicium ou à un atome de carbone d'un groupement hydrocarboné relié à la chaîne organohydrogénopolysiloxane par une liaison Si-C

— et, encapsulées dans ladite matrice, de charges magnétisables présentant une taille généralement inférieure à 30 nm (300 Å), lesdites charges étant revêtues d'un agent dispersant non hydrosoluble.

2. Particules composites magnétisables selon la revendications 1 caractérisées en ce que l'organopolysiloxane SiVi est représenté par la formule suivante :

$$R'' R' R Si O (Si R R''' O)_n (Si R''' R' O)_m Si R R' R''$$

formule dans laquelle :

• les symboles R sont identiques ou différents et représentent un radical alkyle en $C_1$-$C_4$, phényle, trifluoro -3,3,3 propyle ;

• les symboles R' sont identiques ou différents et représentent R ou un radical vinyle, le nombre de radicaux vinyles étant de 2 au moins par macromolécule ;

• les symboles R" sont identiques ou différents et représentent R ou un radical OH

• les symboles R'" sont identiques ou différents et représentent R ou un motif -r-X, où r représente un radical organique bivalent et X un groupe ionogène et/ou réactif non vinylique

• au moins 60% des radicaux représentés par les symboles R, R' et R" sont des radicaux méthyles.

• les symboles n et m peuvent être nuls séparement, R' représentant un radical vinyle si m est nul, et ont une valeur suffisante pour assurer une viscosité du polymère de 20 mPas à 30.000.000 mPas à 25°C et un nombre de motifs ionogènes et/ou réactifs non vinyliques éventuels allant de 1 à 1000, par molécule d'organopolysiloxane SiVi et d'organohydrogénopolysiloxane SiH.

3. Particules composites magnétisables selon la revendication 1, caractérisées en ce que l'organohydrogénopolysiloxane SiH a pour formule :

$$Y R_2 Si O (R R''' Si O)_p (Y R Si O)_q Si R_2 Y$$

formule dans laquelle les symboles R sont identiques ou différents et ont la définition donnée à la revendication 2, avec au moins 80% de radicaux méthyles, le symbole Y représente R ou un atome d'hydrogène, le nombre d'atomes d'hydrogène étant de 3 au moins par molécule de polymère, le symbole R'" a la définition donnée à la revendication 2, les symboles p et q étant tels que ledit polymère SiH présente une viscosité de l'ordre de 5 à 1500 mPas à 25°C, et un nombre de motifs ionogènes et/ou réactifs non vinyliques éventuels allant de 1 à 1000, par molécule d'organohydrogénopolysiloxane SiH et d'organopolysiloxane SiVi.

4. Particules composites magnétisables selon la revendication 2 ou 3 caractérisé en ce que le radical bivalent -r- est un radical alkylène linéaire ou ramifié en $C_1$-$C_{18}$, éventuellement prolongé par 1 à 5 groupes bivalents éthylène amine, par 1 à 50 groupes oxyde d'alkylène en $C_1$-$C_3$, ou par un groupe

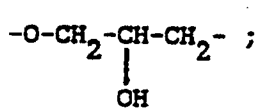

un radical polyoxyalkylène contenant de 1 à 50 chaînons oxyalkylène en $C_1$-$C_3$.

5. Particules composites magnétisables selon l'une quelconques des revendications 2 à 4 caractérisées en ce que le symbole X est une fonction époxy, hydroxy, carboxy, aldéhyde, ester, acétoester, mercapto, mercaptoester, mercaptoalcoxy, amino, alkylamino, dialkylamino, trialkylamino, ammonium quaternaire, aminoalcool, amido, hydrazide, hydrazino, halogénoalkyle en $C_1$-$C_3$, cyano, cyanato,

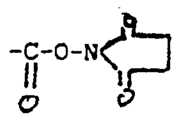 ,

sulfate, sulfonyle, halogénobenzoyle.

6. Particules composites magnétisables selon l'une quelconque des revendications précédentes caractérisées en ce que les charges magnétisables sont constituées de magnétite, hématite, dioxyde de chrome, de ferrites telles que les ferrites de manganèse, nickel, manganèse-zinc, d'alliages de cobalt, nickel, gadolinium, samarium-cobalt.

7. Particules composites magnétisables selon la revendication 4 caractérisées en ce que les charges magnétisables sont constituées de magnétite.

8. Particules composites magnétisables selon l'une quelconque des revendications précédentes caractérisées en ce que leur diamètre va de 0,05 à 3 µm.

9. Particules composites magnétisables selon l'une quelconque des revendications précédentes caractérisées en ce qu'elles contiennent de 0,5 à 50% en poids de charges magnétisables par rapport à la matrice.

10. Particules composites magnétisables selon l'une quelconque des revendications précédentes caractérisées en ce que l'agent dispersant non-hydrosoluble revêtant les charges magnétisables est un acide gras, une amine grasse, un amide gras ou un dispersant siliconé.

11. Dispersions aqueuses de particules composites magnétisables caractérisées en ce qu'elles contiennent de 10 à 70% de leur poids de particules composites magnétisables faisant l'objet de l'une quelconque des revendications précédentes.

12. Procédé de préparation de particules composites magnétisables faisant l'objet de la revendication 1 caractérisé en ce qu'il consiste :

a) à introduire dans un milieu aqueux contenant un tensio-actif un mélange

- d'une solution d'au moins un organopolysiloxane SiVi tel que défini à la revendication 1 dans un solvant organique de point d'ébullition inférieur à 100°C susceptible de former avec l'eau un azéotrope de point d'ébullition inférieur à 100°C
- d'une solution d'au moins un organohydrogénopolysiloxane SiH tel que défini à la revendication 1 dans un solvant organique de point d'ébullition inférieur à 100°C ou susceptible de former avec l'eau un azéotrope de point d'ébullition inférieur à 100°C
- et d'un fluide magnétique constitué de particules magnétisables de taille généralement inférieure à 30 nm (300 Å) en suspension dans un liquide vecteur organique solvant desdits polymères SiVi et SiH et de point d'ébullition inférieur à 100°C ou susceptible de former avec l'eau un azéotrope de point d'ébullition inférieur à 100°C, lesdites particules magnétisables étant revêtues d'un agent dispersant non hydrosoluble,

b) à homogénéiser le milieu obtenu

c) à effectuer une opération d'hydrosilylation en émulsion aqueuse des polymères SiH et SiVi.

d) à éliminer les solvants organiques et le liquide vecteur organique

e) et éventuellement à éliminer l'eau.

13. Procédé selon la revendication 12 caractérisé en ce que l'organopolysiloxane SiVi est représenté par la formule

$$R'' R' R Si O (Si R R''' O)_n (Si R''' R' O)_m Si R R' R''$$

formule dans laquelle :

- les symboles R sont identiques ou différents et représentent un radical alkyle en $C_1$-$C_4$, phényle, trifluoro -3,3,3 propyle ;
- les symboles R' sont identiques ou différents et représentent R ou un radical vinyle, le nombre de radicaux vinyles étant de 2 au moins par macromolécule ;
- les symboles R'' sont identiques ou différents et représentent R ou un radical OH
- les symboles R''' sont identiques ou différents et représentent R ou un motif -r-X, où r représente un radical organique bivalent et X un groupe ionogène et/ou réactif non vinylique

• au moins 60% des radicaux représentés par les symboles R, R' et R" sont des radicaux méthyles.

• les symboles n et m peuvent être nuls séparement, R' représentant un radical vinyle si m est nul, et ont une valeur suffisante pour assurer une viscosité du polymère de 20 mPas à 30.000.000 mPas à 25°C et un nombre de motifs ionogènes et/ou réactifs non vinyliques éventuels allant de 1 à 1000, par molécule d'organopolysiloxane SiVi et d'organohydrogénopolysiloxane SiH.

14. Procédé selon la revendication 12 caractérisé en ce que l'organohydrogénopolysiloxane SiH a pour formule

$$Y R_2 Si O (R R''' Si O)_p (Y R Si O)_q Si R_2 Y$$

formule dans laquelle les symboles R sont identiques ou différents et ont la définition donnée à la revendication 13, avec au moins 80% de radicaux méthyles, le symbole Y représente R ou un atome d'hydrogène, le nombre d'atomes d'hydrogène étant de 3 au moins par molécule de polymère, le symbole R''' a la définition donnée à la revendication 13, les symboles p et q étant tels que ledit polymère SiH présente une viscosité de l'ordre de 5 à 1500 mPas à 25°C, et un nombre de motifs ionogènes et/ou réactifs non vinyliques éventuels allant de 1 à 1000, par molécule d'organohydrogénopolysiloxane SiH et d'organopolysiloxane SiVi.

15. Procédé selon l'une quelconque des revendication 12 à 14 caractérisé en ce que le milieu aqueux contient de 0,5 à 15% en poids de tensio-actif et est mis en oeuvre suivant une quantité correspondant à 0,5 à 60% en poids de tensio-actif par rapport au poids de polymères Si Vi et SiH.

16. Procédé selon l'une quelconque des revendications 13 à 15 caractérisé en ce que le radical bivalent -r- est un radical alkylène linéaire ou ramifié en $C_1$-$C_{18}$, éventuellement prolongé par 1 à 5 groupes bivalents éthylène amine, par 1 à 50 groupes bivalents oxyde d'alkylène en $C_1$-$C_3$ ou par un groupe

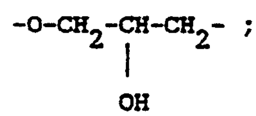

un radical polyoxyalkylène contenant de 1 à 50 chaînons oxyalkylène en $C_1$-$C_3$.

17. Procédé selon l'une quelconque des revendications 13 à 16 caractérisé en ce que le symbole X est un fonction époxy, hydroxy, carboxy, aldéhyde, ester, acétoester, mercapto, mercaptoester, mercaptoalcoxy, amino, alkylamino, dialkylamino, trialkylamino, ammonium quaternaire, aminoalcool, amido, hydrazide, hydrazino, halogénoalkyle en $C_1$-$C_3$, cyano, cyanato,

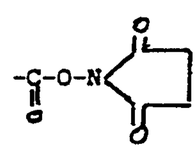

sulfate, sulfonyle, halogénobenzoyle.

18. Procédé selon l'une quelconque des revendications 12 à 17 caractérisé en ce que les charges magnétisables contenues dans le fluide magnétique sont constituées de magnétite, hématite, dioxyde de chrome, de ferrites telles que les ferrites de manganèse, nickel, manganèse-zinc, d'alliages de cobalt, nickel, gadolinium, samarium-cobalt.

19. Procédé selon la revendication 18 caractérisé en ce que les particules magnétisables contenues dans le fluide magnétique sont constituées de magnétite.

20. Procédé selon l'une quelconque des revendications 12 à 19 caractérisé en ce que le fluide magnétique contient de 20 à 60% en poids de charges magnétisables.

21. Procédé selon l'une quelconque des revendications 12 à 20 caractérisé en ce que le poids de charges magnétisables du fluide magnétique correspond à 0,5 à 50% du poids de polymères Si Vi et SiH.

22. Procédé selon l'une quelconque des revendications 12 à 21 caractérisé en ce que le solvant des polymères SiH et Si Vi et le fluide vecteur du fluide magnétique sont semblables ou différents et sont du cyclohexane, du chlorure de méthylène, du benzène, de l'hexane, de l'octane, du toluène, du tétrachlorure de carbone.

23. Procédé selon l'une quelconque des revendications 12 à 22 caractérisé en ce que l'opération d'homogénéisation est réalisée jusqu'à obtenir des gouttelettes de 0,065 à 3,2 µm.

24. Procédé selon l'une quelconque des revendications 12 à 23 caractérisé en ce que l'opération de réticulation est réalisée à une température de 20 à 70°C en présence d'un composé d'un métal du groupe du platine comme catalyseur d'hydrosilylation.

25. Procédé selon la revendication 24 caractérisé en ce que la quantité de catalyseur correspond à 5 à 100 ppm calculé en poids de métal par rapport au poids total de polymères Si Vi et SiH.

26. Procédé selon l'une quelconque des revendications 12 à 25 caractérisé en ce que l'étape d'élimination des solvants précède celle de réticulation.

27. Utilisation des particules composites magnétisables et dispersions de microsphères magnétisables faisant l'objet des revendications 1 à 11 en biologie.

**Patentansprüche**

1. Magnetisierbare Verbundteilchen, die als solche oder in wäßrige Dispersion vorliegen und bestehen aus:

— einer Matrix auf Basis eines vernetzten Organopolysiloxans, das der Hydrosilylierung entstammt von

• zumindest einem Organopolysiloxan (SiVi bezeichnet), welches je Molekül zumindest zwei jeweils an ein Siliciumatom gebundene Vinylgruppen enthält, mit einer Viskosität von etwa 20 bis 30000000 mPas bei 25°C und das gegebenenfalls ionogene und/oder reaktive, nicht-vinylische Gruppen enthält, die an ein Siliciumatom oder an ein Kohlenstoffatom einer Kohlenwasserstoffgruppe, welche an die Organopolysiloxankette über eine Si-C-Bindung geknüpft ist, gebunden sind,

• mit zumindest einem Organohydrogenopolysiloxan (mit SiH bezeichnet), welche je Molekül zumindest drei an ein Siliciumatom gebundene Wasserstoffatome enthält, eine Viskosität von etwa 5 bis 1500 mPas bei 25°C besitzt und gegebenenfalls ionogene und/oder reaktive, nicht-vinylische Gruppen aufweist, welche an ein Siliciumatom oder an ein Kohlenstoffatom einer Kohlenwasserstoffgruppe, gebunden sind, die an die Organohydrogenopolysiloxantette über eine Si-C-Bindung geknüpft sind,

— und, eingekapselt in diese Matrix, aus magnetisierbaren Füllstoffen mit einer Größe im allgemeinen von geringer als 30 nm (300 Å), wobei die Füllstoffe mit einem nicht-wasserlöslichen Dispergiermittel überzogen sind.

2. Magnetisierbare Verbundteilchen gemäß Anspruch 1, dadurch gekennzeichet, daß das Organopolysiloxan SiVi die folgende Formel besitzt :

$$R'' R' R Si O (Si R R''' O)_n (Si R''' R' O)_m Si R R' R''$$

worin

• die Symbole R gleich oder verschieden sind und einen $C_{1-4}$-Alkyl-, Phenyl-, 3,3,3-Trifluorpropylrest bedeuten ;

• die Symbole R' gleich oder verschieden sind und R oder einen Vinylrest wiedergeben, wobei die Anzahl der Vinylgruppen zumindest 2 je Makromolekül ist ;

• die Symbole R'' gleich oder verschieden sind und R oder einen Rest OH bedeuten ;

• die Symbole R''' gleich oder verschieden sind und R oder eine Gruppe -r-X bedeutet, worin r einen zweiwertigen, organischen Rest wiedergibt und X eine ionogene und/oder reaktive, nicht-vinylische Gruppe bedeutet ;

• zumindest 60% der durch die Symbole R, R' und R'' dargestellten Reste Methylreste sind ;

• die Symbole n und m getrennt Null bedeuten können, wobei R' einen Vinylrest bedeutet, wenn m Null ist, und einen ausreichenden Wert aufweisen, um eine Viskosität des Polymeren von 20 mPas bis 30000000 mPas bei 25°C zu gewährleisten, und eine Anzahl an etwaigen ionogenen und/oder reaktiven, nicht-vinylischen Gruppen von 1 bis 1000 je Organopolysiloxanmolekül SiVi und Organohydrogenopolysiloxan SiH besitzen.

3. Magnetisierbare Verbundteilchen gemäß Anspruch 1, dadurch gekennzeichnet, daß das Organohydrogenopolysiloxan SiH die Formel besitzt :

$$Y R_2 Si O (R R''' Si O)_p (Y R Si O)_q Si R_2 Y$$

worin die Symbole R gleich oder verschieden sind und die in Anspruch 2 angegebene Bedeutung aufweisen, wobei zumindest 80% Methylreste sind, das Symbol Y für R oder ein Wasserstoffatom steht, wobei die Anzahl der Wasserstoffatome zumindest 3 je Molekül des Polymeren ist, das Symbol R''' die in Anspruch 2 angegebene Bedeutung hat, die Symbole p und q derart sind, daß das Polymere SiH eine Viskosität in der Größenordnung von 5 bis 1500 mPas bei 25°C aufweist und eine Anzahl an etwaigen ionogenen und/oder reaktiven, nicht-vinylischen Gruppen von 1 bis 1000 je Organohydrogenopolysiloxanmolekül SiH und Organopolysiloxanmolekül

SiVi besitzt.

4. Magnetisierbare Verbundteilchen gemäß Ansprüch 2 oder 3, dadurch gekennzeichnet, daß der zweiwertige Rest -r- ein linearer oder verzweigter $C_{1-18}$-Alkylenrest ist, der gegebenenfalls durch 1 bis 5 zweiwertige Ethylenamingruppen, durch 1 bis 50 $C_{1-3}$-Alkylenoxidgruppen oder durch eine Gruppe

$$-O-CH_2-\underset{OH}{CH}-CH_2-;$$

einen Polyoxyalkylenrest mit 1 bis 50 $C_{1-3}$-Oxyalkylenketten verlängert ist.

5. Magnetisierbare Verbundteilchen gemäß einem der Ansprüche 2 bis 4, dadurch gekennzeichnet, daß das Symbol X eine Epoxy-, Hydroxy-, Carboxy-, Aldehyd-, Ester-, Acetoester-, Mercapto-, Mercaptoester-, Mercaptoalkoxy-, Amino-, Alkylamino-, Dialkylamino-, Trialkylamino-, quaternäre Ammonium-, Aminoalkohol-, Amido-, Hydrazid-, Hydrazino-, $C_{1-3}$-Halogenalkyl-, Cyano-, Cyanato-,

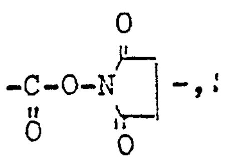

Sulfat-, Sulfonyl- oder Halogenbenzoylfunktion ist.

6. Magnetisierbare Verbundteilchen gemäß einem der vorhergehenden Ansprüche, dadurch gekennzeichnet, daß die magnetisierbaren Füllstoffe aus Magnetit, Hämatit, Chromdioxid, Ferriten, wie den Ferriten von Mangan, Nickel, Mangan-Zink, Legierungen von Kobalt, Nickel, Gadolinium, Samarium-Kobalt bestehen.

7. Magnetisierbare Verbundteilchen gemäß Anspruch 4, dadurch gekennzeichnet, daß die magnetisierbaren Füllstoffe aus Magnetit bestehen.

8. Magnetisierbare Verbundteilchen gemäß einem der vorhergehenden Ansprüche, dadurch gekennzeichnet, daß deren Durchmesser 0,05 bis 3 μm beträgt.

9. Magnetisierbare Verbundteilchen gemäß einem der vorhergehenden Ansprüche, dadurch gekennzeichnet, daß sie 0,5 bis 50 Gew.% magnetisierbare Füllstoffe, bezogen auf die Matrix, enthalten.

10. Magnetisierbare Verbundteilchen gemäß einem der vorhergehenden Ansprüche, dadurch gekennzeichnet, daß das die magnetisierbaren Füllstoffe umhüllende, nicht-wasserlösliche Dispergiermittel eine Fettsäure, ein Fettamin, ein Fettamid oder ein Silicondispergiermittel ist.

11. Wäßrige Dispersionen magnetisierbarer Verbundteilchen, dadurch gekennzeichnet, daß sie 10 bis 70% ihres Gewichts an magnetisierbaren Verbundteilchen gemäß einem der vorhergehenden Ansprüche enthalten.

12. Verfahren zur Herstellung von magnetisierbaren Verbundteilchen gemäß Anspruch 1, dadurch gekennzeichnet, daß es darin besteht:

(a) in ein wäßriges, ein oberflächenaktives Mittel enthaltendes Milieu ein Gemisch einzubringen von

- einer Lösung von zumindest einem Organopolysiloxan SiVi, wie in Anspruch 1 definiert, in einem organischen Lösungsmittel mit einem Siedepunkt von geringer als 100°C, das befähigt ist, mit Wasser ein Azeotrop mit einem Siedepunkt von geringer als 100°C zu bilden,
- einer Lösung von mindestens einem Organohydrogenopolysiloxan SiH, wie in Anspruch 1 definiert, in einem organischen Lösungsmittel mit einem Siedepunkt von geringer als 100°C oder befähigt, mit Wasser ein Azeotrop mit einem Siedepunkt von geringer als 100°C zu bilden,
- und einer magnetischen Flüssigkeit, bestehend aus magnetisierbaren Teilchen mit einer Größe von im allgemeinen geringer als 30 nm (300 Å) in Suspension in einer organischen Vektorflüssigkeit, welche die Polymeren SiVi und SiH löst, mit einem Siedepunkt von geringer als 100°C oder befähigt, mit Wasser ein Azeotrop mit einem Siedepunkt von geringer als 100°C zu bilden, wobei die magnetisierbaren Teilchen von einem nicht-wasserlöslichen Dispergiermittel umhüllt sind,

(b) das erhaltene Milieu zu homogenisieren,

(c) ein Hydrosilylierungsverfahren in wäßriger Emulsion der Polymeren SiH und SiVi durchzuführen,

(d) die organischen Lösungsmittel und die organische Vektorflüssigkeit zu entfernen,

(e) und gegebenenfalls das Wasser zu entfernen.

13. Verfahren gemäß Anspruch 12, dadurch gekennzeichnet, daß das Organopolysiloxan SiVi die folgende Formel besitzt:

$$R'' R' R Si O (Si R R''' O)_n (Si R''' R' O)_m Si R R' R''$$

worin

- die Symbole R gleich oder verschieden sind und einen $C_{1-4}$-Alkyl-, Phenyl-, 3,3,3-Trifluorpropylrest bedeuten ;
- die Symbole R′ gleich oder verschieden sind und R oder einen Vinylrest wiedergeben, wobei die Anzahl der Vinylgruppen zumindest 2 je Makromolekül ist ;
- die Symbole R″ gleich oder verschieden sind und R oder einen Rest OH bedeuten ;
- die Symbole R‴ gleich oder verschieden sind und R oder eine Gruppe -r-X bedeuten, worin r einen zweiwertigen, organischen Rest wiedergibt und X eine ionogene und/oder reaktive, nicht-vinylische Gruppe bedeutet ;
- zumindest 60% der durch die Symbole R, R′ und R″ dargestellten Reste Methylreste sind ;
- die Symbole n und m getrennt Null bedeuten können, wobei R′ einen Vinylrest bedeutet, wenn m Null ist, und einen ausreichenden Wert aufweisen, um eine Viskosität des Polymeren von 20 mPas bis 30000000 mPas bei 25°C zu gewährleisten, und eine Anzahl von etwaigen ionogenen und/oder reaktiven, nicht-vinylischen Gruppen von 1 bis 1000 je Organopolysiloxanmolekül SiVi und Organohydrogenopolysiloxanmolekül SiH besitzen.

14. Verfahren gemäß Anspruch 12, dadurch gekennzeichnet, daß das Organohydrogenpolysiloxan SiH die Formel

$$Y\,R_2\,Si\,O\,(R\,R'''\,Si\,O)_p\,(Y\,R\,Si\,O)_q\,Si\,R_2\,Y$$

besitzt, worin die Symbole R gleich oder verschieden sind und die in Anspruch 13 angegebene Bedeutung haben, wobei zumindest 80% Methylreste sind, das Symbol Y für R oder ein Wasserstoffatom steht, wobei die Anzahl der Wasserstoffatome zumindest 3 je Molekül des Polymeren ist, das Symbol R‴ die in Anspruch 13 angegebene Bedeutung besitzt, die Symbole p und q derart sind, daß das Polymere SiH eine Viskosität in der Größenordung von 5 bis 1500 mPas bei 25°C aufweist und eine Anzahl an etwaigen ionogenen und/oder reaktiven, nicht-vinylischen Gruppen von 1 bis 1000 je Organohydrogenopolysiloxanmolekül SiH und Organopolysiloxanmolekül SiVi besitzt.

15. Verfahren gemäß einem der Ansprüche 12 bis 14, dadurch gekennzeichnet, daß das wäßrige Milieu 0,5 bis 15 Gew.% oberflächenaktives Mittel enthält und in einer Menge entsprechend 0,5 bis 60 Gew.% oberflächenaktives Mittel, bezogen auf das Gewicht der Polymeren SiVi und SiH, eingesetzt wird.

16. Verfahren gemäß einem der Ansprüche 13 bis 15, dadurch gekennzeichnet, daß der zweiwertige Rest -r- ein linearer oder verzweigter $C_{1-18}$-Alkylenrest ist, der gegebenenfalls durch 1 bis 5 zweiwertige Ethylenamingruppen, durch 1 bis 50 zweiwertige $C_{1-3}$-Alkylenoxidgruppen oder durch eine Gruppe

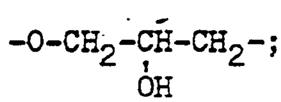

einen Polyoxyalkylenrest mit 1 bis 50 $C_{1-3}$-Oxyalkylenketten verlängert ist.

17. Verfahren gemäß einem der Ansprüche 13 bis 16, dadurch gekennzeichnet, daß das Symbol X eine Epoxy-, Hydroxy-, Carboxy-, Aldehyd-, Ester-, Acetoester-, Mercapto-, Mercaptoester-, Mercaptoalkoxy-, Amino-, Alkylamino-, Dialkylamino-, Trialkylamino-, quaternäre Ammonium-, Aminoalkohol-, Amido-, Hydrazid-, Hydrazino-, $C_{1-3}$-Halogenalkyl-, Cyano-, Cyanato-,

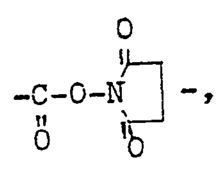

Sulfat-, Sulfonyl-, Halogenbenzoylfunktion ist.

18. Verfahren gemäß einem der Ansprüche 12 bis 17, dadurch gekennzeichnet, daß die in dem magnetischen Fluid enthaltenen, magnetisierbaren Füllstoffe aus Magnetit, Hämatit, Chromdioxid, Ferriten, wie den Ferriten von Mangan, Nickel, Mangan-Zink, Legierungen von Kobalt, Nickel, Gadolinium, Samarium-Kobalt bestehen.

19. Verfahren gemäß Anspruch 18, dadurch gekennzeichnet, daß die in dem magnetischen Fluid enthaltenen, magnetisierbaren Füllstoffe aus Magnetit bestehen.

20. Verfahren gemäß einem der Ansprüche 12 bis 19, dadurch gekennzeichnet, daß das magnetische Fluid 20 bis 60 Gew.% magnetisierbare Füllstoff enthält.

21. Verfahren gemäß einem der Ansprüche 12 bis 20, dadurch gekennzeichnet, daß das Gewicht der magnetisierbaren Füllstoffe des magnetischen Fluids 0,5 bis 50% des Gewichts der Polymeren SiVi und SiH entspricht.

22. Verfahren gemäß einem der Ansprüche 12 bis 21, dadurch gekennzeichnet, daß das Lösungsmittel der Polymeren SiH und SiVi und das Vektorfluid des magnetischen Fluids ähnlich oder verschieden sind und Cyclohehan, Methylenchlorid, Benzol, Hexan, Octan, Toluol oder Tetrachlorkohlenstoff darstellen.

23. Verfahren gemäß einem der Ansprüche 12 bis 22, dadurch gekennzeichnet, daß das Homogenisierungsverfahren bis zur Erzielung von Tröpfchen von 0,065 bis 3,2 µm durchgeführt wird.

24. Verfahren gemäß einem der Ansprüche 12 bis 23, dadurch gekennzeichnet, daß das Vernetzungsverfahren bei einer Temperatur von 20 bis 70°C in Gegenwart einer Verbindung eines Metalls der Platingruppe als Hydrosilylierungskatalysator durchgeführt wird.

25. Verfahren gemäß Anspruch 24, dadurch gekennzeichnet, daß die Menge des Katalysators 5 bis 100 ppm, berechnet als Gewicht des Metalls, bezogen auf das Gesamtgewicht der Polymeren SiVi und SiH, entspricht.

26. Verfahren gemäß einem der Ansprüche 12 bis 25, dadurch gekennzeichnet, daß die Stufe der Entfernung der Lösungsmittel derjenigen der Vernetzung vorangeht.

27. Verwendung der magnetisierbaren Verbundteilchen und der Dispersionen der magnetisierbaren Mikrokügelchen der Ansprüche 1 bis 11 in der Biologie.

**Claims**

1. Magnetisable composite particles present as such or in aqueous dispersion and consisting :

— of a matrix based on a crosslinked organopolysiloxane derived from the hydrosilylation

— of at least one organopolysiloxane (called SiVi) containing at least two vinyl groups per molecule, each bonded to a silicon atom, having a viscosity of the order of 20 to 30,000,000 mPas at 25°C and bearing, if desired, ion-forming and/or nonvinyl reactive units bonded to a silicon atom or to a carbon atom of a hydrocarbon group linked by an Si-C bond to the organopolysiloxane chain

— with at least one organohydropolysiloxane (called SiH) containing at least three hydrogen atoms per molecule, each bonded to a silicon atom, having a viscosity of the order of 5 to 1500 mPas at 25°C, and bearing ; if desired, ion-forming and/or nonvinyl reactive units bonded to a silicon atom or to a carbon atom of a hydrocarbon group linked by an Si-C bond to the organohydropolysiloxane chain

— and, encapsulated in the said matrix, magnetisable fillers generally smaller than 30 nm (300 A) in size, the said fillers being coated with a water-insoluble dispersing agent.

2. Magnetisable composite particles according to Claim 1, characterised in that the organopolysiloxane SiVi is denoted by the following formula :

$$R'' R' R Si O (Si R R''' O)_n (Si R''' R' O)_m Si R R' R''$$

in which formula :

— the symbols R are identical or different and denote a $C_1$-$C_4$ alkyl, phenyl, or 3,3,3-trifluoropropyl radical;

— the symbols R' are identical or different and denote R or a vinyl radical, the number of vinyl radicals being at least 2 per macromolecule ;

— the symbols R" are identical or different and denote R or an OH radical

— the symbols R‴ are identical or different and denote R or a unit -r-X, where r denotes a divalent organic radical and X an ion-forming and/or nonvinyl reactive group

— at least 60% of the radicals denoted by the symbols R, R' and R" are methyl radicals.

— the symbols n and m may be zero separately, R' denoting a vinyl radical if m is zero, and have a sufficient value to ensure a polymer viscosity of 20 mPas to 300,000,000 mPas at 25°C and a number of ion-forming and/or nonvinyl reactive units which may be present ranging from 1 to 1000 per molecule of organopolysiloxane SiVi and of organohydropolysiloxane SiH.

3. Magnetisable composite particles according to Claim 1, characterised in that the organohydropolysiloxane SiH has the formula :

$$Y R_2 Si O (R R''' Si O)_p (Y R Si O)_q Si R_2 Y$$

in which formula the symbols R are identical or different and have the definition given in Claim 2, with at least 80% of methyl radicals, the symbol Y denotes R or a hydrogen atom, the number of hydrogen atoms being at least 3 per molecule of polymer, the symbol R''' has the definition given in Claim 2, the symbols p and q being such that the said polymer SiH has a viscosity of the order of 5 to 1500 mPas at 25°C, and a number of ion-forming and/or nonvinyl reactive units which may be present ranging from 1 to 1000 per molecule of organohydropolysiloxane SiH and organopolysiloxane SiVi.

4. Magnetisable composite particles according to Claim 2 or 3, characterised in that the divalent radical -r- is a linear or branched $C_1$-$C_{18}$ alkylene radical extended, if desired, by 1 to 5 divalent ethylene amine groups, by 1 to 50 $C_1$-$C_3$ alkylene oxide groups or by an

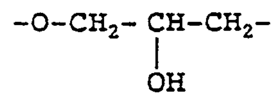

group, or a polyoxyalkylene radical containing from 1 to 50 $C_1$-$C_3$ oxyalkylene chain units.

5. Magnetisable composite particles according to any one of Claims 2 to 4, characterised in that the symbol X is an epoxy, hydroxyl, carboxyl, aldehyde, ester, acetoester, mercapto, mercaptoester, mercaptoalkoxy, amino, alkylamino, dialkylamino, trialkylamino, quaternary ammonium, aminoalcohol, amido, hydrazide, hydrazino, $C_1$-$C_3$ haloalkyl, cyano, cyanato,

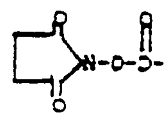 ,

sulphate, sulphonyl or halobenzoyl functional group.

6. Magnetisable composite particles according to any one of the preceding claims, characterised in that the magnetisable fillers consist of magnetite, haematite, chromium dioxide, ferrites such as manganese, nickel and manganese-zinc ferrites, and alloys of cobalt, nickel, gadolinium and samarium-cobalt.

7. Magnetisable composite particles according to Claim 4, characterised in that the magnetisable fillers consist of magnetite.

8. Magnetisable composite particles according to any one of the preceding claims, characterised in that their diameter ranges from 0.05 to 3 µm.

9. Magnetisable composite particles according to any one of the preceding claims, characterised in that they contain from 0.5 to 50% by weight of magnetisable fillers relative to the matrix.

10. Magnetisable composite particles according to any one of the preceding claims, characterised in that the water-insoluble dispersing agent coating the magnetisable fillers is a fatty acid, a fatty amine, a fatty amide or a silicone dispersant.

11. Aqueous dispersions of magnetisable composite particles, characterised in that they contain from 10 to 70% of their weight of magnetisable composite particles forming the subject of any one of the preceding claims.

12. Process for the preparation of magnetisable composite particles forming the subject of Claim 1, characterised in that it consists :

a) in introducing into an aqueous medium containing a surfactant a mixture

— of a solution of at least one organopolysiloxane SiVi as defined in Claim 1 in an organic solvent with a boiling point below 100°C capable of forming with water an azeotrope with a boiling point below 100°C

— of a solution of at least one organohydropolysiloxane SiH as defined in Claim 1 in an organic solvent with a boiling point below 100°C or capable of forming with water an azeotrope with a boiling point below 100°C

— and of a magnetic fluid consisting of magnetisable particles generally below 30 nm (300 A) in size in suspension in an organic carrier liquid dissolving the said polymers SiVi and SiH and with a boiling point below 100°C or capable of forming with water an azeotrope with a boiling point below 100°C, the said magnetisable particles being coated with a water-insoluble dispersing agent,

b) in homogenising the mixture obtained,

c) in performing a hydrosilylation operation in an aqueous emulsion of the polymers SiH and SiVi,

d) in removing the organic solvents and the organic carrier liquid, and

e) in removing the water, if appropriate.

13. Process according to Claim 12, characterised in that the organopolysiloxane SiVi is denoted by the formula

$$R'' R' R Si O (Si R R''' O)_n (Si R''' R' O)_m Si R R' R''$$

in which formula

— the symbols R are identical or different and denote a $C_1$-$C_4$ alkyl, phenyl or 3,3,3-trifluoropropyl radical;
— the symbols R' are identical or different and denote R or a vinyl radical, the number of vinyl radicals being at least 2 per macromolecule ;
— the symbols R" are identical or different and denote R or an OH radical ;
— the symbols R‴ are identical or different and denote R or a unit -r-X, where r denotes a divalent organic radical and X an ion-forming and/or nonvinyl reactive group
— at least 60% of the radicals denoted by the symbols R, R' and R" are methyl radicals.
— the symbols n and m may be zero separately, R' denoting a vinyl radical if m is zero, and have a sufficient value to ensure a polymer viscosity of 20 mPas to 30,000,000 mPas at 25°C and a number of ion-forming and/or nonvinyl reactive units which may be present ranging from 1 to 1000 per molecule of organopolysiloxane SiVi and of organohydropolysiloxane SiH.

14. Process according to Claim 12, characterised in that the organohydropolysiloxane SiH has the formula

$$Y R_2 Si O (R R''' Si O)_p (Y R Si O)_q Si R_2 Y$$

in which formula the symbols R are identical or different and have the definition given in Claim 13, with at least 80% of methyl radicals, the symbol Y denotes R or a hydrogen atom, the number of hydrogen atoms being at least 3 per polymer molecule, the symbol R‴ has the definition given in Claim 13, the symbols p and q being such that the said polymer SiH has a viscosity of the order of 5 to 1500 mPas at 25°C, and a number of ion-forming and/or nonvinyl reactive units which may be present ranging from 1 to 1000 per molecule of organohydropolysiloxane SiH and of organopolysiloxane SiVi.

15. Process according to any one of Claims 12 to 14, characterised in that the aqueous medium contains from 0.5 to 15% by weight of surfactant and is employed in a quantity corresponding to 0.5 to 60% by weight of surfactant relative to the weight of polymers SiVi and SiH.

16. Process according to any one of Claims 13 to 15, characterised in that the divalent radical -r- is a linear or branched $C_1$-$C_{18}$ alkylene radical extended, if desired, by 1 to 5 divalent ethylene amine groups, by 1 to 50 divalent $C_1$-$C_3$ alkylene oxide groups or by an

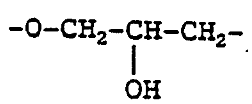

group, or a polyoxyalkylene radical containing from 1 to 50 $C_1$-$C_3$ oxyalkylene chain units.

17. Process according to any one of Claims 13 to 16, characterised in that the symbol X is an epoxy, hydroxyl, carboxyl, aldehyde, ester, acetoester, mercapto, mercaptoester, mercaptoalkoxy, amino, alkylamino, dialkylamino, trialkylamino, quaternary ammonium, aminoalcohol, amido, hydrazide, hydrazino, $C_1$-$C_3$ haloalkyl, cyano, cyanato,

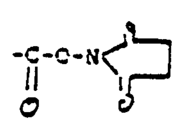

,

sulphate, sulphonyl or halobenzoyl functional group.

18. Process according to any one of Claims 12 to 17, characterised in that the magnetisable fillers present in the magnetic fluid consist of magnetite, haematite, chromium dioxide, ferrites such as manganese, nickel and manganese-zinc ferrites or alloys of cobalt, nickel, gadolinium or samarium-cobalt.

19. Process according to Claim 18, characterised in that the magnetisable particles contained in the magnetic fluid consist of magnetite.

20. Process according to any one of Claims 12 to 19, characterised in that the magnetic fluid contains from 20 to 60% by weight of magnetisable fillers.

21. Process according to any one of Claims 12 to 20, characterised in that the weight of magnetisable fillers

in the magnetic fluid corresponds to 0.5 to 50% of the weight of polymers SiVi and SiH.

22. Process according to any one of Claims 12 to 21, characterised in that the solvent of the polymers SiH and SiVi and the carrier fluid of the magnetic fluid are similar or different and are cyclohexane, methylene chloride, benzene, hexane, octane, toluene or carbon tetrachloride.

23. Process according to any one of Claims 12 to 22, characterised in that the homogenisation operation is carried out until droplets from 0.065 to 3.2 μm are obtained.

24. Process according to any one of Claims 12 to 23, characterised in that the crosslinking operation is carried out at a temperature of 20 to 70°C in the presence of a compound of a platinum group metal as a hydrosilylation catalyst.

25. Process according to Claim 24, characterised in that the quantity of catalyst corresponds to 5 to 100 ppm, calculated as the weight of metal relative to the total weight of polymers SiVi and SiH.

26. Process according to any one of Claims 12 to 25, characterised in that the solvent removal stage precedes that of crosslinking.

27. Use of the magnetisable composite particles and dispersions of magnetisable microspheres forming the subject of Claims 1 to 11 in biology.